# EUROPEAN PATENT SPECIFICATION

(11) **EP 0 637 997 B1**
(45) Date of publication and mention of the grant of the patent: **01.07.1998**
(21) Application number: 93910889.0
(22) Date of filing: 29.04.1993
(51) Int. Cl.: B01L 3/00, G01N 33/00

(54) **ANALYSIS BASED ON FLOW RESTRICTION**
ANALYSE BEI MESSUNG DES DURCHFLUSSWIDERSTANDES
ANALYSE FONDEE SUR UN RESSERREMENT DU SYSTEME D'ECOULEMENT

(30) Priority: 01.05.1992 US 877536; 01.05.1992 US 877661; 01.05.1992 US 877662; 01.05.1992 US 877701; 01.05.1992 US 877702
(43) Date of publication of application: 15.02.1995
(73) Proprietor: TRUSTEES OF THE UNIVERSITY OF PENNSYLVANIA, Philadelphia, PA 19104 (US)
(72) Inventor: WILDING, Peter, Paoli, PA 19301 (US); KRICKA, Larry, J., Berwyn, PA 19312 (US)
(74) Representative: Armitage, Ian Michael
(86) International application number: US9304016
(87) International publication number: WO9322054

(56) References cited:
- EP-A- 0 483 117
- WO-A-91/15750
- US-A- 4 790 640

## Description

### Background of the Invention

This invention relates generally to methods and apparatus for conducting analyses. More particularly, the invention relates to the design and construction of small, typically single-use, modules capable of rapidly determining the presence of an analyte in a fluid sample.

In recent decades the art has developed a very large number of protocols, test kits, and cartridges for conducting analyses on biological samples for various diagnostic and monitoring purposes. Immunoassays, agglutination assays, and analyses based on polymerase chain reaction, various ligand-receptor interactions, and differential migration of species in a complex sample all have been used to determine the presence or concentration of various biological compounds or contaminants, or the presence of particular cell types.

Recently, small, disposable devices have been developed for handling biological samples and for conducting certain clinical tests. Shoji et al. reported the use of a miniature blood gas analyser fabricated on a silicon wafer. Shoji et al., Sensors and Actuators, 15:101-107 (1988). Sato et al. reported a cell fusion technique using micromechanical silicon devices. Sato et al., Sensors and Actuators, A21-A23:948-953 (1990). Ciba Corning Diagnostics Corp. (USA) has manufactured a microprocessor-controlled laser photometer for detecting blood clotting.

Micromachining technology originated in the microelectronics industry. Angell et al., Scientific American, 248:44-55 (1983). Micromachining technology has enabled the manufacture of microengineered devices having structural elements with minimal dimensions ranging from tens of micrometers (the dimensions of biological cells) to nanometers (the dimensions of some biological macromolecules). This scale is referred to herein as "mesoscale". Most experiments involving mesoscale structures have involved studies of micromechanics, i.e., mechanical motion and flow properties. The potential capability of mesoscale structures has not been exploited fully in the life sciences.

Brunette (Exper. Cell Res., 167:203-217 (1986) and 164:11-26 (1986)) studied the behavior of fibroblasts and epithelial cells in grooves in silicon, titanium-coated polymers and the like. McCartney et al. (Cancer Res., 41:3046-3051 (1981)) examined the behavior of tumor cells in grooved plastic substrates. LaCelle (Blood Cells, 12:179-189 (1986)) studied leukocyte and erythrocyte flow in microcapillaries to gain insight into microcirculation. Hung and Weissman reported a study of fluid dynamics in micromachined channels, but did not produce data associated with an analytic device. Hung et al., Med. and Biol. Engineering, 9:237-245 (1971); and Weissman et al., Am. Inst. Chem. Eng. J., 17:25-30 (1971). Columbus et al. utilized a sandwich composed of two orthogonally orientated v-grooved embossed sheets in the control of capillary flow of biological fluids to discrete ion-selective electrodes in an experimental multi-channel test device. Columbus et al., Clin. Chem., 33:1531-1537 (1987). Masuda et al. and Washizu et al. have reported the use of a fluid flow chamber for the manipulation of cells (e.g. cell fusion). Masuda et al., Proceedings IEEE/IAS Meeting, pp. 1549-1553 (1987); and Washizu et al., Proceedings IEEE/IAS Meeting pp. 1735-1740 (1988). The art has not fully explored the potential of using mesoscale devices for the analyses of biological fluids and detection of microorganisms.

EP-A-0483117 discloses a capillary flow device including a chamber, a capillary and a reagent, such as a labelled antibody, which reacts with an analyte to produce a detectable signal, such as light absorption or emission, or a flow rate change. In the device, the capillary provides the sole driving force for movement of liquid through the device without the use of pumps or the like.

The current analytical techniques utilized for the detection of microorganisms are rarely automated, usually require incubation in a suitable medium to increase the number of organisms, and invariably employ visual and/or chemical methods to identify the strain or sub-species. The inherent delay in such methods frequently necessitates medical intervention prior to definitive identification of the nature of an infection. In industrial, public health or clinical environments, such delays may have serious consequences. There is a need for convenient systems for the rapid detection of microorganisms.

In some aspects, the invention provides analytical systems that can analyze microvolumes of sample and produce analytical results rapidly. In other aspects, easily mass produced, disposable, small (e.g., less than 1 cc in volume) devices are provided having mesoscale functional elements capable of rapid, automated analyses of preselected molecular or cellular analytes, in a range of applications. In further aspects the invention provides a family of such devices that individually can be used to implement a range of rapid tests, e.g., tests for bacterial or viral infection, sperm motility, blood parameters, contaminants in food, water, or body fluids, and the like. In further aspects, the invention provides a family of analytical assay protocols for detecting the presence of an analyte wherein the information indicative of a positive assay is obtained by measuring directly or indirectly alteration of flow properties of fluid flowing through a restricted passage.

### Summary of the Invention

Accordingly, in two aspects the present invention provides methods and apparatus for detecting the presence of an analyte in a fluid sample as set out in claims 1 and 20. In one embodiment, the invention provides a device comprising a solid substrate, typically on the order of a few millimeters thick and approximately a 0.2 to 2.0 centimeters square, microfabricated to define a sample inlet port and a mesoscale flow system. The invention provides a method wherein a sample fluid is passed through the mesoscale flow system, and the analyte induced restriction or blockage of flow through the system is detected as a positive indication of the presence of the analyte. The mesoscale flow system includes a primary sample flow channel, extending from the inlet port, and a fractal region, in fluid communication with the primary flow channel, comprising bifurcations leading to plural secondary flow channels. The term "mesoscale" is used herein to define flow passages having cross-sectional dimensions on the order of approximately 0.1 µm to 500 µm, with preferred widths on the order of 2.0 to 500 µm, more preferably 3 - 100 µm. For many applications, channels of 5 - 50 µm widths will be useful. Chambers in the substrates often may have larger dimensions, e.g., widths and lengths of 1 - 5 mm. Preferred depths are on the order of 0.1 to 100 µm, typically 2 - 50 µm.

The fractal region typically further comprises junctions, in fluid communication with the secondary flow channels, leading to a third flow channel. The fractal region may comprise equal numbers of bifurcations and junctions disposed serially along the direction of flow. Preferably, but not necessarily, the branching channels in the fractal region progressively decrease in cross-sectional area at each bifurcation and increase at each junction. The fractal flow region is very sensitive to the flow properties of a sample. Means may be provided in the device for inducing flow of the sample through the flow system. Means also may be provided in the device for detecting changes in flow properties, such as restriction or blockage of flow, induced by the presence of an analyte. The devices and methods of the invention may be used to implement a variety of automated, sensitive and rapid tests including analyses for the presence of particular types of cells or macromolecules, for monitoring reactions or cell growth, or for conducting sperm motility testing.

Generally, as disclosed herein, the solid substrate comprises a chip containing the mesoscale flow system. The mesoscale flow system may be designed and fabricated from silicon and other solid substrates using established micromachining methods. The mesoscale flow systems in the devices may be constructed by microfabricating flow channels and one or more fractal regions into the surface of the substrate, and then adhering a cover, e.g., a transparent glass cover, over the surface. The devices typically are designed on a scale suitable to analyze microvolumes (<5 µL) of sample, introduced into the flow system through an inlet port defined, e.g., by a hole communicating with the flow system through the substrate or the cover. Analytes present in very low concentrations (e.g., nanogram quantities) can be rapidly detected (<10 minutes). After an assay is complete, the devices can be discarded.

In one embodiment, a specific binding moiety may be provided in the mesoscale flow system, e.g., in the fractal region, to enhance restriction or blockage of sample flow through the flow system. The binding moieties may comprise particles which bind with a component of the sample to induce detectable particle agglomeration. Optionally, the binding moiety may be immobilized on the internal surfaces of the mesoscale flow system, so that binding induces stenosis of the passage.

The chips typically will be used with an appliance which contains a nesting site for holding the chip, and which mates one or more input ports on the chip with one or more flow lines in the appliance. After a fluid sample, e.g., a biological fluid sample, suspected to contain a particular analyte, such as a cellular contaminant, or toxin, is applied to the inlet port of the substrate, the chip is placed in the appliance and a pump, e.g., in the appliance, is actuated to force the sample through the flow system. Alternatively, a sample may be injected into the chip by the appliance. The sample also may enter the flow system simply by capillary action through an inlet port.

The presence of a preselected analyte in a fluid sample may be detected by sensing analyte*-*induced changes in sample fluid flow properties, such as changes in the pressure or electrical conductivity, at different points in the flow system. In one embodiment, analyte induced restriction or blockage of flow in the mesoscale flow system, e.g., in the fractal region, may be detected by pressure detectors, e.g., in the appliance used in combination with the device. In another embodiment, analyte-induced changes in conductivity in a region of the flow system caused by introduction of a sample fluid may be readily detected through electrical conductivity sensors in contact with the flow system. For example, the presence of analyte may cause clogging of a restricted flow passage, and beyond the passage, the absence of liquid can be detected by measuring conductivity. The appliance also may include electrical contacts in the nesting region which mate with contacts integrated into the structure of the chip to, e.g., receive electrical signals indicative of a pressure reading, conductivity, or the like, sensed in some region of the flow system to indicate flow restriction, as a positive indication of the presence of the analyte.

Analyte induced changes in flow properties of a sample fluid also may be detected optically, e.g., through a transparent or translucent window, such as a transparent cover over the flow system, or through a translucent section of the substrate itself. The appliance may include sensing equipment, such as a spectrophotometer, capable of detecting analyte induced changes in flow properties of a sample through an optical window in a chip.

The devices of the invention can be adapted to perform a wide range of biological tests. Some of the features and benefits of the devices are summarized in Table 1. A device may include two or more separated flow systems, e.g., fed by a common inlet port, each with different binding moieties in, e.g., different fractal detection regions, to enable the detection of two or more analytes simultaneously. The device may also comprise a control flow system so that data from the sample region and the control region may be detected and compared. The devices can provide rapid clinical tests for the detection of, e.g., pathogenic bacteria, or viruses, or to test, e.g., the motility of a sperm sample. The invention provides methods and devices for use in a wide range of possible assays. Assays may be completed rapidly, and at the conclusion of the assay the chip can be discarded, which advantageously prevents contamination between samples, entombs potentially biologically hazardous material, and provides an inexpensive, microsample analysis.

**TABLE 1**

| Feature | Benefit |
|---|---|
| Flexibility | No limits to the number of chip designs or applications available. |
| Reproducible | Allows reliable, standardized, mass production of chips. |
| Low Cost Production | Allows competitive pricing with existing systems. Disposable nature for single-use processes. |
| Small Size | No bulky instrumentation required. Lends itself to portable units and systems designed for use in non-conventional lab environments. Minimal storage and shipping costs. |
| Microscale | Minimal sample and reagent volumes required. Reduces reagent costs, especially for more expensive, specialized test procedures. Allows simplified instrumentation schemes. |
| Sterility | Chips can be sterilized for use in microbiological assays and other procedures requiring clean environments. |
| Sealed System | Minimizes biohazards. Ensures process integrity. |
| Multiple Circuit Capabilities | Can perform multiple processes or analyses on a single chip. Allows panel assays. |
| Multiple Detector Capabilities | Expands capabilities for assay and process monitoring to virtually any system. Allows broad range of applications. |
| Reuseable Chips | Reduces per process cost to the user for certain applications. |

### Brief Description of the Drawings

FIGURE 1 is a magnified plan view of device 10 according to the invention that comprises substrate 14 microfabricated with ports 16, mesoscale flow channel 20, and a fractally bifurcating system of flow channels 40.

FIGURE 2 is a longitudinal cross sectional view of the device shown in Figure 1.

FIGURE 3 is a perspective view of the device of Figure 1.

FIGURE 4 is a schematic cross sectional view of an analytical device 10 nested within an appliance 50, which is used to support the device 10 and to regulate and detect the pressure of sample fluids in device 10.

FIGURE 5 is a schematic plan view of a substrate 14 microfabricated with a fractally bifurcating system of flow channels 40 symmetrically disposed on the substrate, and tapering to a narrower diameter towards the center of the fractal system.

FIGURE 6 is a schematic plan view of device 10 that includes substrate 14 microfabricated with entry ports 16, mesoscale flow channel 20, and a fractally bifurcating system of flow channels 40, provided with beads 42 to enhance flow restriction and agglomeration in the fractal.

FIGURE 7 is a schematic longitudinal cross-sectional view of a device according to the invention which includes electrical conductors 17 and 18 for measuring conductivity of fluids in the device.

FIGURE 8 is a perspective view of the device shown in Figure 7.

FIGURE 9 is a schematic plan view of a multitest apparatus constructed in accordance with the invention.

FIGURE 10 is a schematic plan view of an analytical device fabricated with a series of mesoscale chambers suitable for implementing a variety of functions including cell sorting, cell lysing, PCR analysis, and detection of PCR products in the fractal region 40.

FIGURE 11 is a schematic plan view of device 10 according to the invention that includes substrate 14 microfabricated with ports 16, mesoscale flow channels 20, and a pair of fractal flow channels 40.

FIGURE 12 is a schematic perspective view of an apparatus 60 used in combination with device 10 for viewing the contents of device 10.

FIGURE 13 is a schematic cross sectional view of the apparatus 60 of Figure 12.

Like reference characters in the respective drawn figures indicate corresponding parts.

### Detailed Description

The invention provides methods and apparatus for detecting the presence of an analyte in a fluid sample. In one embodiment, the invention provides a device comprising a solid substrate, typically on the order of a few millimeters thick and 0.2 to 2.0 centimeters square, microfabricated to define a sample inlet port and a mesoscale flow system. A sample fluid is passed through the mesoscale flow system, and the analyte induced restriction or blockage of flow through the system is detected as a positive indication of the presence of the analyte.

Analytical devices having mesoscale flow channels and fractal regions can be designed and fabricated in large quantities from a solid substrate material. They can be sterilized easily. Silicon is a preferred substrate material because of the well-developed technology permitting its precise and efficient fabrication, but other materials may be used including polymers such as polytetrafluoroethylenes. The sample inlet and other ports, the mesoscale flow system, including the sample flow channel(s), the fractal region(s), and other functional elements, may be fabricated inexpensively in large quantities from a silicon substrate by any of a variety of micromachining methods known to those skilled in the art. The micromachining methods available include film deposition processes such as spin coating and chemical vapor deposition, laser fabrication or photolithographic techniques such as UV or X-ray processes, or etching methods which may be performed by either wet chemical processes or plasma processes. (See, e.g., Manz et al., Trends in Analytical Chemistry, 10: 144-149 (1991)).

Flow channels of varying widths and depths can be fabricated with mesoscale dimensions. The silicon substrate containing a fabricated mesoscale flow channel may be covered and sealed with a thin anodically bonded glass cover. Other clear or opaque cover materials may be used. Alternatively, two silicon substrates can be sandwiched, or a silicon substrate can be sandwiched between two glass covers. The use of a transparent cover results in a window which facilitates dynamic viewing of the channel contents, and allows optical probing of the mesoscale flow system either visually or by machine. Other fabrication approaches may be used. In one embodiment, electron micrographs of biological structures such as circulatory networks may be used as masks for fabricating mesoscale flow systems on the substrate. Mesoscale flow systems may be fabricated in a range of sizes and conformations. The flow system comprises a fractal region including bifurications leading to plural secondary channels. In the devices, flow restriction in the mesoscale flow system serves as a positive indicator of the presence of an analyte.

The capacity of the devices is very small and therefore the amount of sample fluid required for an analysis is low. For example, in a 1 cm x 1 cm silicon substrate, having on its surface an array of 500 grooves which are 10 micrometers wide x 10 micrometers deep x 1 cm (10⁴ micrometers) long, the volume of each groove is 10⁻³ µL and the total volume of the 500 grooves is 0.5 µL. The low volume of the mesoscale flow systems allows assays to be performed on very small amounts of a liquid sample (<10µL). The volume of the flow system typically will be <5µL, and the volume of individual channels, chambers, or other functional elements are often less than 1 µl, e.g., in the nanoliter or picoliter range. The mesoscale flow systems of the devices may be microfabricated with microliter volumes, or alternatively nanoliter volumes or less, which advantageously limits the amount of sample and/or reagent fluids required for the assay.

An important consequence and advantage of employing flow channels having mesoscale dimensions is that alterations in the flow properties of macromolecules, particles, and cells entrained or dissolved in aqueous liquids within the channels is easily influenced by stenosis, i.e., narrowing of the flow channels, and easily detected. The provision of the fractal region serves to simplify alteration in flow. Thus, for example, a sample suspected to be contaminated with bacteria can be cultured in the device and the presence of a multiplicity of the organism can be detected by determining whether fluid can be forced through the system at a given pressure. Where no bacteria is present, fluid would flow easily; a large number of cells would serve to partially or totally occlude the fractal region. As another example, accretion of macromolecules onto specific binding proteins immobilized on the walls of the flow channel is sufficient to inhibit liquid flow through the channel provided its dimensions are small enough. In still another example, the presence of a target polynucleotide in a polynucleotide sample may be indicated by flowing the contents of a chamber after a suitable number of PCR cycles through a fractal region, as the viscosity of a solution laden with a large amount of polynucleotides will be larger than a solution of nucleotides.

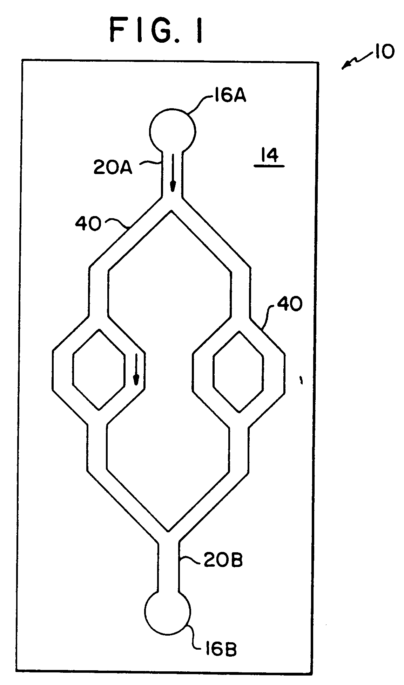

In one embodiment, illustrated schematically in Figures 1, 2 and 3, the device 10 may include a silicon substrate 14 microfabricated with ports 16, primary sample flow channel 20A, and a fractal system of flow channels 40. The ports may be microfabricated with mesoscale or larger dimensions. The fractal region 40 in this case comprises equal numbers of bifurcations and junctions, disposed serially along the direction of flow through the fractal region, leading to a third flow channel 20B. The substrate 14 is covered with a clear glass or plastic window 12 to close the channels. In operation, a fluid sample enters the device through inlet port 16A and flow channel 20A, and then flows through the fractal region 40 to flow channel 20B and port 16B. The fractal region 40 is very sensitive to the flow properties of a sample. Restriction or blockage of flow of a sample through the fractal region 40 can serve as an indicator of the presence of an analyte in the sample and may be detected, e.g., optically through the window 12.

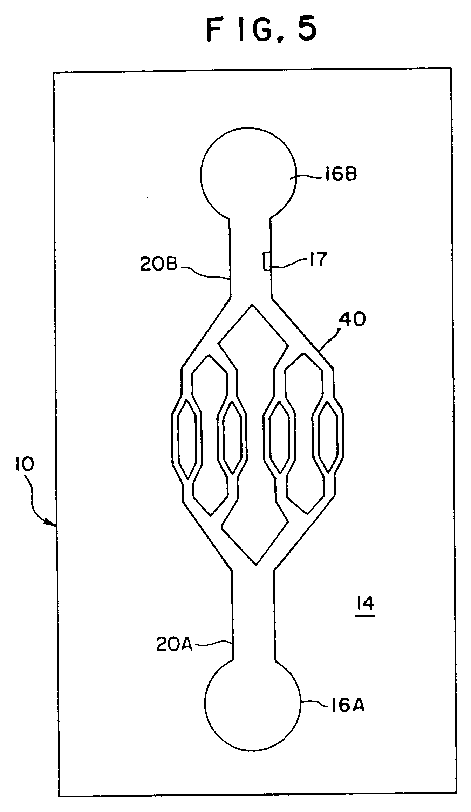

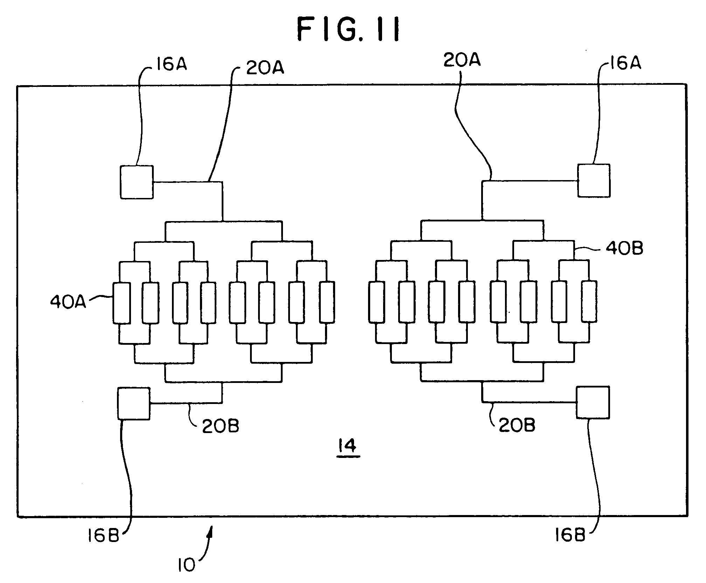

In another embodiment, the fractal system 40 may be fabricated on a silicon substrate with reduced dimensions at each bifurcation, providing sequentially narrower flow channels, as illustrated schematically in Figure 5. Figure 5 shows device 10, which comprises substrate 14 microfabricated with fractal flow channels 40, which have a reduced cross-sectional area relative to the primary flow channel 20A and the third flow channel 20B. In operation, a sample fluid enters the device 10 through inlet port 16A and channel 20A, and then flows through the fractal region 40 to flow channel 20B and port 16B. Fluid flow through this fractal region 40 is very sensitive to changes in fluid viscosity and to the development of flow restriction caused, for example, by the proliferation of cells, or the agglomeration of cells, particles, or macromolecular complexes that may be present in a sample. The fractal system may be microfabricated with a complex series of bifurcations, as illustrated schematically in Figure 11, to enhance sensitivity to flow restriction. Device 10 in Figure 11 includes a pair of fractally bifurcating flow channels 40A and 40B. The fractal flow channel 40A is constructed with sequentially narrower flow channels towards the center of the fractal, thereby enhancing sensitivity to flow restriction.

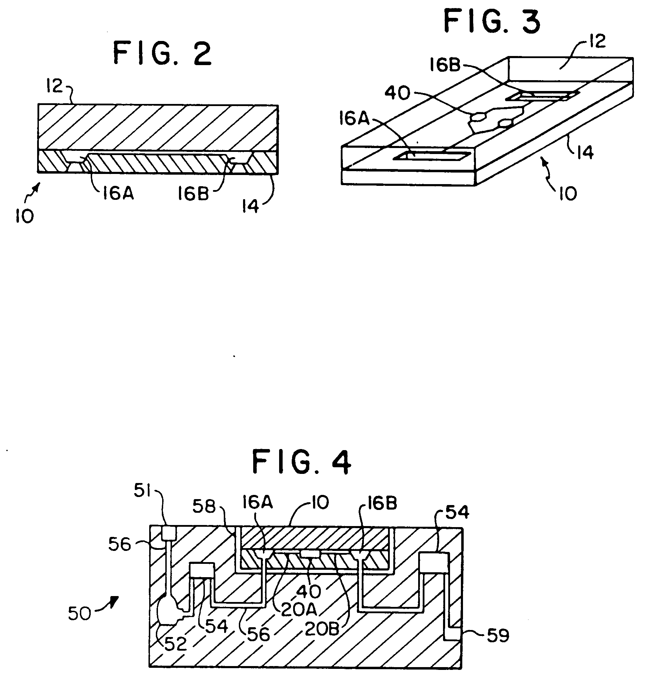

The analytical devices containing the mesoscale flow system can be used in combination with an appliance for delivering and receiving fluids to and from the devices, such as appliance 50 shown schematically in Figure 4, which incorporates a nesting site 58 for holding the device 10, and for registering ports, e.g., ports 16 on the device 10, with a flow line 56 in the appliance. After a fluid sample suspected to contain a particular analyte is applied to the inlet port 51 of the appliance, pump 52 is actuated to force the sample into port 16A of device 10, flow channel 20A, and the fractal region 40. Alternatively, the sample may be injected into the device, or may enter the flow system simply by capillary action. In one embodiment, the flow systems of the devices may be filled to a hydraulically full volume, and the appliance may be utilized to direct the flow of fluid in the mesoscale flow system by means, e.g., of valves located in the device or the appliance.

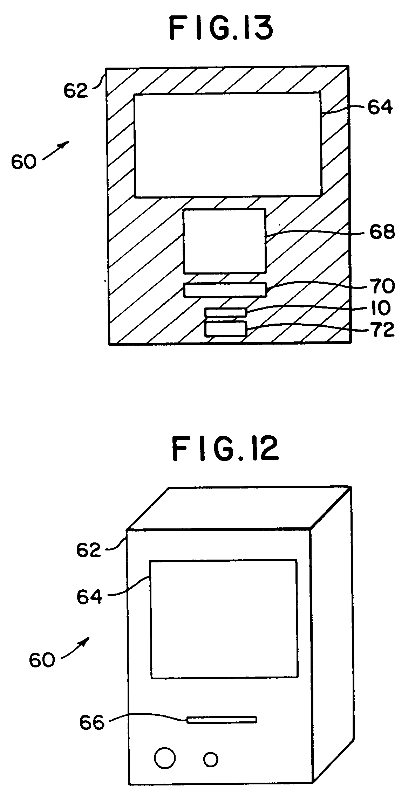

The analytical devices also may be utilized in combination with an appliance for viewing the contents of the mesoscale channels in the devices. The appliance in one embodiment may comprise a microscope for viewing the contents of the mesoscale channels in the devices. In another embodiment, a camera may be included in the appliance, as illustrated in the appliance 60 shown schematically in Figures 12 and 13. The appliance 60 is provided with a housing 62, a viewing screen 64 and a slot 66 for inserting a chip into the appliance. As shown in cross section in Figure 13, the appliance 60 also includes a video camera 68, an optical system 70, and a tilt mechanism 72 for holding device 10, and allowing the placement and angle of device 10 to be adjusted manually. The optical system 70 may include a lens system for magnifying the channel contents, as well as a light source. The video camera 68 and screen 64 allow analyte induced change in sample fluid properties, such as flow properties or color, to be monitored visually, and optionally recorded using the appliance.

Changes in sample flow properties in the flow system, induced by the presence of an analyte in the sample, can be detected by any of a number of methods including monitoring the pressure or electrical conductivity of sample fluids in selected regions of the flow system in the device as disclosed herein. Analyte induced changes in flow properties also may be detected by optical detection through a transparent cover or a translucent section of the substrate itself, either visually or by machine. Devices such as valves, mesoscale pressure sensors, and other mechanical sensors can be fabricated directly on the silicon substrate and can be mass-produced according to well established technologies. Angell et al., Scientific American, 248:44-55 (1983). Pressure sensors and other detection means also may be provided in an appliance utilized in combination with the device.

In one embodiment, analyte induced flow restriction can be detected by monitoring the pressure of sample fluids entering and exiting the mesoscale flow system. Figure 4 shows schematically, as an example, device 10, which is nested within appliance 50, which includes two pressure detectors 54 for detecting flow pressure of fluids entering and exiting device 10 through ports 16. Alternatively, a mesoscale pressure sensor may be fabricated directly on the silicon substrate and connected via electrical contacts to the appliance. Angell et al., Scientific American, 248:44-55 (1983). Analyte induced changes in flow properties in the flow system, such as flow restriction, thus may be detected as a pressure change indicative of a positive result. Other detectors may be utilized, such as conventional flow detectors. The movement of magnetic beads entrained in the fluid can be detected easily as an indication of flow restriction.

In another embodiment, electrical conductors may be fabricated in the substrate of the devices to enable transmission of signals indicative of alterations in fluid flow properties, induced by the presence of the analyte, and sensed in different regions of the flow system. Electrical conductors in the substrate may be mated through contacts to the electrical conductors in an appliance, used in combination with the device. The electrical conductors in the device carry signals from pressure or electrical conductivity sensors enabling the detection of the conductivity or pressure of fluid in the flow systems.

For example, in the device 10, illustrated schematically in Figure 5, analyte induced clogging of the fractal region 40, which blocks flow from inlet port 16A to outlet port 16B, may be detected by a conventional conductivity probe 17 whose output is indicative of the presence or absence of aqueous fluid in the outflow channel. The conductivity or other probe could also be fabricated within the fractal region 40. The substrate may be microfabricated with a control region such that output from the sample flow region and the control region may be detected and compared, thereby enhancing the accuracy of the assay.

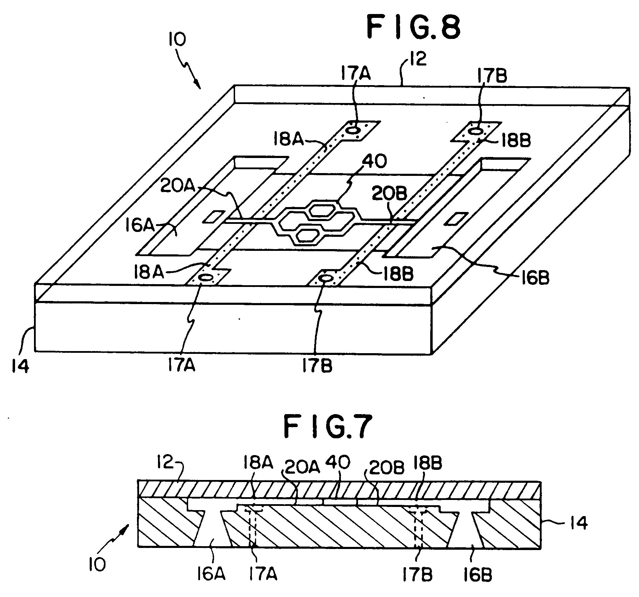

In another embodiment, the flow properties between sample fluid entering and exiting the flow system can be detected and compared in order to detect analyte induced changes in flow properties of a sample. In one embodiment, the conductivity may be measured in the device 10 shown schematically in Figures 7 and 8. Device 10 includes the silicon substrate 14 on which are microfabricated inlet ports 16 and flow channel 20. The substrate is covered by a translucent window 12.

In operation, a sample fluid enters device 10 through port 16A and sample channel 20A, and then flows through the fractal region 40 to channel 20B and port 16B. Device 10 is microfabricated with electrical conductor 18A in electrical contact with fluid channel 20A, for detecting the conductivity of fluid centering the fractal region region 40. The device also includes electrical conductor 18B, in electrical contact with flow channel 20B, for detecting the conductivity of fluid exiting the fractal region 40. The conductors 18 are connected to contacts 17 which extend through to the bottom of the substrate. The contacts 17 can be fabricated by known techniques, e.g., by thermal gradient zone melting. (See Zemel et al., in: Fundamentals and Applications of Chemical Sensors, D. Schuetzle and R. Hammerle, Eds., ACS Symposium Series 309, Washington, DC, 1986, p. 2.) Device 10 may be nested in an appliance such as appliance 50, shown in Figure 4, capable of detecting conductivity changes through the contacts 17. Changes in conductivity can be correlated with changes in fluid properties, such as fluid pressure, induced by the presence of an analyte, in the fluid sample. Blockage in the fractal will prevent liquid from reaching channel 20B, and the conductivity across the gap in conductor 18B will be low.

Analyte induced changes in flow properties of a sample in the flow systems, such as flow restriction, also may be detected optically, e.g., with a microscope, through a transparent cover over the flow system, or through a transparent region of the substrate itself. The appliance may include sensing equipment, such as a spectrophotometer, to assist in the optical detection of changes in flow properties due to the presence of the analyte.

In one embodiment, the mesoscale flow system, e.g., the fractal region, may comprise a binding moiety, capable of binding the analyte, thereby to enhance flow restriction. Optionally, the binding moiety may be immobilized on the surface of the flow channels, or on a solid phase reactant such as a bead. The binding moiety, may comprise, e.g., an antigen binding protein, a DNA probe, or one of a ligand/receptor pair. The binding moiety may also comprise a crosslinker, such as a chemical reagent or a protein, capable of crosslinking of a specific cell subpopulation.

The binding moiety may be immobilized on the surface of the mesoscale flow channels by, e.g., physical absorption onto the channel surfaces, or by chemical activation of the surface and subsequent attachment of biomolecules to the activated surface. Techniques available in the art may be utilized for the chemical activation of silaceous channel surfaces, and for the subsequent attachment of a binding moiety to the surfaces. (See, e.g., Haller in: Solid Phase Biochemistry, W.H. Scouten, Ed., John Wiley, New York, pp 535-597 (1983); and Mandenius et al., Anal. Biochem., 137:106-114 (1984), and Anal. Biochem., 170:68-72 (1988)). The detection of a cellular or chemical analyte can be implemented by selecting the appropriate binding moiety. Flow restriction may be enhanced by the binding of the analyte to the binding moiety, immobilized on the surface of the mesoscale flow system, i.e., by the build-up of a macromolecular surface layer on the surface of the flow system.

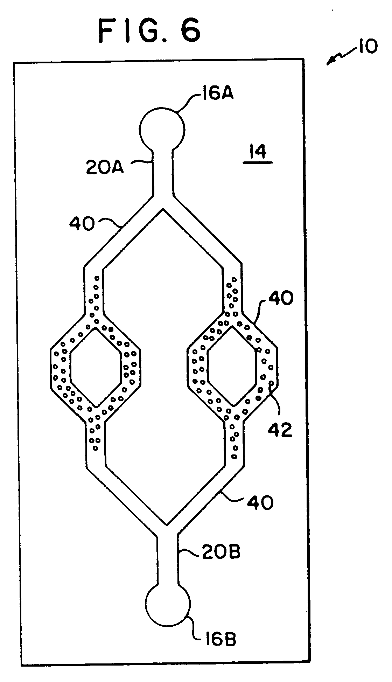

In one embodiment, the binding moiety may comprise a particle capable of inducing detectable agglomeration of an analyte in the mesoscale flow system. As illustrated in device 10, shown schematically in Figure 6, particles 42 coated with binding protein specific for a given analyte may be provided in the fractal region 40 to promote analyte-induced agglomeration of fluid in the fractal region. For example, a binding moiety such as an antibody may be immobilized on an inert bead, and may be utilized to induce agglomeration. Agglomeration in the fractal region may be detected optically through a window, e.g., disposed over the fractal region. Agglomeration may also be detected by, e.g., detecting pressure or conductivity changes of the sample fluid as noted below.

In order to enhance the accuracy of an assay, the substrate may be fabricated to include a control region in the flow system, e.g., a region which is identical in geometry to the test region, but does not include binding moieties. Sample directed to both the detection and control regions exhibit different flow properties which may be detected and compared.

In one embodiment, the devices provide a mesoscale fractal flow system, which readily allows the growth of organisms in a culture to be monitored on the basis of flow restriction, due to changes in fluid viscosity. The fractal region may include an extensive series of equal numbers of bifurcations and junctions disposed serially along the direction of flow of sample through the region, as schematically illustrated in Figure 11. Flow restriction may be detected, e.g., optically, after a short incubation. The presence and growth of an organism in a sample will influence the flow characteristics within the fractal. One or more sensors, such as pressure or conductivity sensors, may be utilized to detect pressure changes due to changes in fluid properties caused by the presence of an organism in the fractal region.

In another embodiment, the migration of sperm in the mesoscale flow systems of the devices, e.g., in a fractal region, can serve as an indication of sperm motility. The substrate may be disposed, e.g., in an appliance, at an angle with respect to a horizontal plane, to provide an incline for the travel of a sperm sample, to further enhance the detection of the motility. Reagents capable of binding to a sperm may be provided in the flow system. The devices may be utilized to assess, e.g., a spermicidal agent, the binding properties of a sperm sample, or to conduct sperm counts.

The devices may be used to implement a variety of automated, sensitive and rapid analyses based on flow restriction including analyses of cells or macromolecules, or for monitoring cell culture growth. The devices may be fabricated with two or more mesoscale flow systems which comprise, e.g., two or more different fractal regions, containing, e.g., binding moieties for different analytes, allowing two or more assays to be conducted simultaneously. At the conclusion of the assay the devices typically are discarded. The use of disposable devices eliminates contamination among samples. The sample at all times can remain entombed, and the low volume simplifies waste disposal.

The invention will be understood further from the following nonlimiting examples.

### Example 1

Sperm motility is tested in the chip 10 shown schematically in Figure 5. A sample of semen (<2µL) is placed on a glass microscope slide, and the chip 10 is placed on top of the semen sample such that the port 16A is positioned on the semen sample. The progress of individual spermatozoa into port 16A, through channel 20A and fractal region 40 is monitored using a microscope. The experimental results may be compared with results previously established for a healthy sperm sample to provide a test of sperm motility.

### Example 2

The growth of an organism is monitored in the device shown schematically in Figure 5. The fractal pattern of mesoscale flow paths 40 in the substrate 14 are filled via inlet port 16A with 2 µL of a mixture of growth medium which has been inoculated with a sample of a test specimen. The device is sealed and incubated for 60 minutes at 37°C. Growth is detected by visual inspection using a microscope or by determining the flow properties of the channel system, e.g., via the electrical conductivity probe 17. The absence of flow indicates growth and consequent blockage of the fractal system.

### Example 3

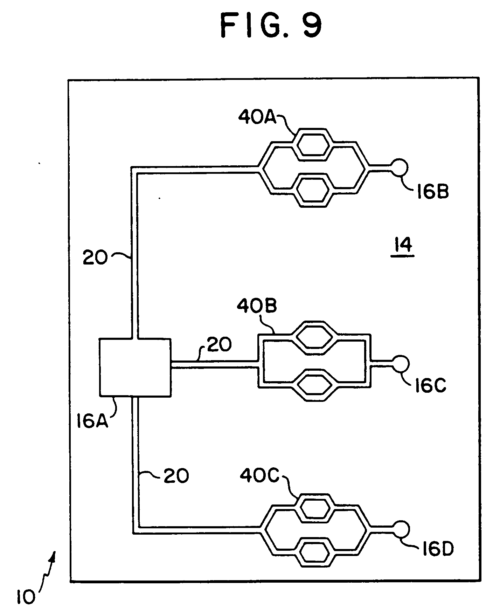

Sperm functions are tested on the microfabricated solid substrate 14 shown in Figure 9. A sperm sample is added to the inlet port 16A and then flows through the mesoscale flow channel 20 to the detection chambers 40A, 40B and 40C. Fractal detection chamber 40A provides a test for leucocytes and comprises immobilized antibody to common leukocyte antigen. Fractal detection chamber 40B provides a test for sperm antibodies and contains immobilized antibody to human IgG, IgA or IgM. Fractal detection chamber 40C provides a test for acrosome reaction and contains fluorescein labeled lectin. Flow restriction due to agglutination in the chambers may be detected, e.g., by optical detection through a glass cover disposed over the substrate. After the assay is complete, the device is discarded.

### Example 4

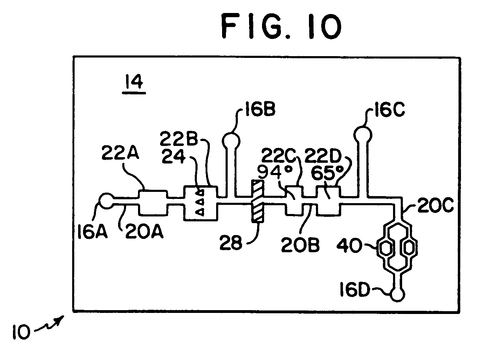

Figure 10 depicts schematically a device 10 including substrate 14 used to detect the presence of a target nucleic acid within a subpopulation of cells in a mixture in a biological fluid sample. Microfabricated on device 10 is a mesoscale flow path 20 which includes a cell separation chamber 22A, a cell lysis chamber 22B, a filter region 28, a polymerase chain reaction (PCR) chamber comprising sections 22C and 22D, and a fractal detection region 40. The mesoscale flow system 20 is also provided with fluid entry/exit ports 16A, 16B, 16C and 16D. The device is used in combination with an appliance, such as appliance 50, shown in Figure 4. The appliance is provided with fluid paths mated to ports 16 in the device, and valves allowing the ports 16 to be mechanically closed and opened. The appliance also includes pump 52 for regulating the flow of sample fluid through the device. The appliance further includes means for heating the PCR reaction chamber sections 22C and 22D in the device.

Initially, valves in the appliance are used to close ports 16C and 16D, while ports 16A and 16B are open. A sample containing a mixture of cells is directed to the sample inlet port 16A by the pump 52 in the appliance, and flows through the mesoscale flow path 20 to separation chamber 22A. Chamber 22A contains binding moieties immobilized on the wall of the chamber which selectively bind to a surface molecule on a desired type of cell in the sample. Remaining cellular components exit the substrate via port 16B. After binding of the desired cell population in chamber 22A, flow with buffer is continued, to wash and assure isolation of the cell population. Next port 16B is closed and 16C is opened. Flow is then increased sufficiently to dislodge the immobilized cells. Flow is continued, forcing cells through membrane piercing protrusions 24 in chamber 22B, which tear open the cells releasing intracellular material.

Sample flow continues past filter 28, which filters off large cellular membrane components and other debris, to mesoscale PCR chamber section 22C, which is connected to PCR chamber section 22D by flow channel 20B. Taq polymerase, primers and other reagents required for the PCR assay next are added to section 22D through port 16C from a mated port and flow path in the appliance, permitting mixing of the intracellular soluble components from the separated subpopulation of cells and the PCR reagents. With port 16A closed, a pump in the appliance connected via port 16B is used to cycle the PCR sample and reagents through flow channel 20B between sections 22C and 22D, set at 94°C and 65°C respectively, to implement plural polynucleotide melting and polymerization cycles, allowing the amplification of product polynucleotide.

The valves in the appliance next are used to close port 16C and to open port 16D. The pump in the appliance connected to port 16B is then used to direct the amplified polynucleotide isolated from the cell population to the fractal detection region 40. Flow restriction in the fractal region 40, caused by the presence of amplified polynucleotide product, serves as a positive indicator of the presence of the target DNA or RNA in the cells, and is detected optically through a glass cover disposed over the detection region.

### Example 5

Experiments were performed in mesoscale flow channels testing the sperm motility of human semen samples. In a sperm motility test, a fractal channel (40 µm wide, 20 µm deep) in a glass-silicon chip was filled with Human Tubal Fluid (HTF) medium (Irvine Scientific, Santa Ana, CA) containing 0.5% BSA (HTF-BSA). A sample of semen (<2µL) was placed on a glass microscope slide and the chip placed on top of the semen sample such that the entrance to the channel was positioned on the semen sample. The progress of individual spermatozoa into the channel and along its length to the exit hole was monitored using a microscope, and recorded using a TV camera and video recorder. Sperm were observed migrating through the tortuous fractal pathway (a total of 9 right angle turns, e.g., the device of Figure 11) from the entry to the center of the channel. The experiment was repeated using a fractal channel which was 20 µm deep, but which was reduced in width at each bifurcation (40, 30, 25, 20, and 10 µm) and then increased in width (20, 25, 30, 40 µm). Again sperm migrated to the center of the fractal channel.

The bi-directional motility of a sperm sample was also examined. A fractal channel was filled with HTF-BSA medium, and semen were introduced simultaneously via the holes at each end of the channel. Sperm were observed migrating towards the center of the channel (or fractal channel) and eventually passing as they migrated towards the hole at the opposite end of the channel.

## Claims

1. A device for detecting the presence of an analyte in a fluid sample, the device comprising:
a solid substrate microfabricated to define:
a sample inlet port; and
a mesoscale flow system comprising:
a primary sample flow channel extending from said inlet port; and
a fractal region, in fluid communication with said primary flow channel; and,
means for detecting a flow property of a fluid sample in said flow system as an indication of the presence of an analyte in the fluid sample; characterised by said fractal region comprising multiple bifurcations each bifurcation leading to plural, distinct secondary flow channels for amplifying the effect of occlusion of said flow system or increase in viscosity within said substrate.

2. The device of claim 1 wherein said fractal region further comprises junctions in fluid communication with said secondary flow channels leading to a third flow channel in said mesoscale flow system.

3. The device of claim 1 or claim 2 further comprising pump means for inducing flow of said sample through said mesoscale flow system.

4. The device of claim 2 or claim 3 wherein said fractal region comprises equal numbers of bifurcations and junctions disposed serially along the direction of flow of a sample through said region.

5. The device of any one of claims 2 to 4 wherein each said secondary channel in said fractal region has a reduced cross-sectional area relative to said primary flow channel and said third flow channel.

6. The device of any one of the preceding claims wherein said means for detecting comprises means for detecting a fluid flow property in said third flow channel.

7. The device of any one of claims 1 to 5 wherein said means for detecting comprises means for detecting and comparing a fluid flow property in said primary sample flow channel with a fluid flow property in said third flow channel.

8. The device of any one of the preceding claims wherein said means for detecting comprises:
(a) an electrical detection means; or,
(b) a magnetic detection means; or,
(c) means defining an optical path to said fractal region; or,
(d) means for detecting the growth of an organism in said flow system.

9. The device of any one of the preceding claims wherein fluid pressure or fluid conductivity is detected.

10. The device claim 1 further comprising a binding moiety disposed within said fractal region for binding a component of said sample.

11. The device of claim 10 wherein said binding moiety comprises particles which bind with a component of said sample to induce particle agglomeration.

12. The device of any one of the preceding claims wherein said substrate defines a plurality of said flow systems.

13. The device of any one of the preceding claims wherein the sample is a sperm sample and wherein flow of sperm through the fractal region provides an indication of sperm motility.

14. The device of claim 13, wherein said substrate is disposed at an angle with respect to a horizontal plane.

15. The device of any one of the preceding claims wherein said solid substrate comprises microfabricated silicon.

16. The device of any one of the preceding claims, further comprising an appliance for use in combination with said substrate, said appliance comprising:
means for holding said substrate;
fluid input means interfitting with the inlet port on said substrate; and
pump means for passing fluid through the flow system of said substrate when it is held in said holding means.

17. The device of any one of claims 1 to 7 and 9 to 16 wherein said means for detecting comprises an appliance for use in combination with said substrate, said appliance comprising:
means for holding said substrate; and
optical means for viewing the contents of said mesoscale flow system in said substrate.

18. The device of claim 17 wherein said optical means comprises magnifying optics and a video camera, and wherein said appliance further comprises:
a tilt mechanism for manually adjusting the angle and location of the device; and
a video screen for viewing the contents of said flow system.

19. The device of any one of the preceding claims wherein said flow system includes a control fractal region permitting comparison of flow of said sample in said fractal region and said control region.

20. A method for detecting the presence of an analyte in a fluid sample, the method comprising the steps of:
(i) providing a device comprising:
a solid substrate microfabricated to define:
a sample inlet port; and
a mesoscale flow system comprising:
a primary sample flow channel extending from said inlet port; and
a fractal region, in fluid communication with said primary flow channel, comprising multiple bifurcations each bifurcation leading to plural, distinct secondary flow channels for amplifying the effect of occlusion of said flow system or increase in viscosity within said substrate;
(ii) passing a fluid sample suspected to contain an analyte through said fractal region in said mesoscale flow system;
(iii) detecting the restriction or blockage of flow of the fluid sample through said system; and
(iv) correlating the detected restriction or blockage of flow, or lack thereof, to the presence or absence of an analyte in said sample.

21. The method of claim 20, wherein said flow system comprises a binding moiety, capable of binding said analyte in said sample, to promote said restriction or blockage of flow through said system.

22. The method of claim 21 wherein said binding moiety is disposed on particles which bind with said analyte in said sample to induce particle agglomeration, thereby to promote said restriction or blockage of flow.

23. The method of claim 21 or claim 22 wherein said analyte is a cell population in said sample;
said binding moiety comprises a crosslinker of cells in said population; and
said restriction of flow is caused by crosslinker-induced cell aggregation.

24. The method of any one of claims 21 to 23 wherein said binding moiety is immobilized within said flow system.

25. The method of any one of claims 20 to 23 wherein flow is restricted by the build-up of a macromolecular surface layer on a surface of said flow system.

26. The method of any one of claims 20 to 25 wherein said fractal region, in the device provided in step (i), further comprises junctions in fluid communication with said secondary flow channels leading to a third channel in said flow system.

27. The method of any one of claims 20 to 26 wherein, in step (iii), restriction or blockage is detected electrically or optically.

28. The method of any one of claims 20 to 27 wherein said fractal region further comprises a binding moiety for binding a component of said sample.

29. The method of any one of claims 20 to 28 wherein said fractal region contains particles which bind with a component of said sample to induce particle agglomeration.

30. The method of any one of claims 20 to 29 wherein said substrate, provided in step (i), further comprises a control region in fluid communication with said sample inlet port; and
wherein, in step (iii), flow of said sample in said fractal region and said control region is detected and compared.

31. The method of any one of claims 20 to 30 wherein the analyte comprises a replicable procaryotic organism; and
wherein, in step (iii), the restriction or blockage of flow of said organism through said flow system serves as an indication of the presence of said organism.

32. The method of any one of claims 20 to 31 wherein, in step (ii), a pump is utilized to deliver said sample through said flow system.

## Patentansprüche

1. Vorrichtung zum Nachweis der Gegenwart eines Analyten in einer Fluidprobe, wobei die Vorrichtung umfaßt:
ein festes Substrat, das durch Mikrofabrikation so hergestellt ist, daß es definiert:
eine Probeneinlaßöffnung; und
ein Meso*-*Scale*-*Durchflußsystem, umfassend:
einen primären Probenströmungskanal, der sich von der Einlaßöffnung weg erstreckt; und
einen Fraktalbereich in Fluidkommunikation mit dem primären Strömungskanal; und
Mittel zur Bestimmung einer Flußeigenschaft einer Fluidprobe im Durchflußsystem als Indikator für die Gegenwart eines Analyten in der Fluidprobe; dadurch gekennzeichnet, daß der Fraktalbereich mehrere Gabelungen umfaßt, wobei jede Gabelung zu mehreren unterschiedlichen sekundären Strömungskanälen führt, um die Verschlußwirkung des Durchflußsystems zu verstärken oder die Viskosität innerhalb des Substrats zu erhöhen.

2. Vorrichtung nach Anspruch 1, worin der Fraktalbereich weiters Verbindungen in Fluidkommunikation mit den sekundären Strömungskanälen umfaßt, die zu einem dritten Strömungskanal im Meso-Scale-Durchflußsystem führen.

3. Vorrichtung nach Anspruch 1 oder 2, die weiters Pumpmittel umfaßt, um ein Fließen der Probe durch das Meso-Scale-Durchflußsystem herbeizuführen.

4. Vorrichtung nach Anspruch 2 oder 3, worin der Fraktalbereich die gleiche Anzahl an Gabelungen und Verbindungen umfaßt, die in Strömungsrichtung einer Probe durch den Bereich hindurch seriell angeordnet sind.

5. Vorrichtung nach einem der Ansprüche 2 bis 4, worin jeder sekundäre Kanal im Fraktalbereich relativ zum primären Strömungskanal und dem dritten Strömungskanal eine verringerte Querschnittsfläche aufweist.

6. Vorrichtung nach einem der vorangegangenen Ansprüche, worin das Detektionsmittel Mittel zum Bestimmen einer Flußeigenschaft des Fluids im dritten Strömungskanal umfaßt.

7. Vorrichtung nach einem der Ansprüche 1 bis 5, worin das Detektionsmittel Mittel umfaßt, um eine Flußeigenschaft des Fluids im primären Probenströmungskanal zu bestimmen und mit einer Flußeigenschaft des Fluids im dritten Strömungskanal zu vergleichen.

8. Vorrichtung nach einem der vorangegangenen Ansprüche, worin das Detektionsmittel umfaßt:
(a) eine elektrisches Detektionsmittel; oder
(b) ein magnetisches Detektionsmittel; oder
(c) Mittel, die einen Lichtweg zum Fraktalbereich hin definieren; oder
(d) Mittel zur Bestimmung des Wachstums eines Organismus im Strömungssystem.

9. Vorrichtung nach einem der vorangegangenen Ansprüche, worin der Fluiddruck oder die Fluidleitfähigkeit bestimmt wird.

10. Vorrichtung nach Anspruch 1 die weites eine innerhalb des Fraktalbereichs angeordnete Bindungsgruppe zur Bindung einer Komponente der Probe umfaßt.

11. Vorrichtung nach Anspruch 10, worin die Bindungsgruppe Teilchen umfaßt, die sich mit einer Komponente der Probe verbinden, sodaß Teilchenagglomeration herbeigeführt wird.

12. Vorrichtung nach einem der vorangegangenen Ansprüche, worin das Substrat mehrere Strömungssysteme definiert.

13. Vorrichtung nach einem der vorangegangenen Ansprüche, worin die Probe eine Spermaprobe ist und worin das Fließen von Sperma durch den Fraktalbereich hindurch einen Indikator für die Spermienbeweglichkeit darstellt.

14. Vorrichtung nach Anspruch 13, worin das Substrat in einem bestimmten Winkel zu einer horizontalen Ebene angeordnet ist.

15. Vorrichtung nach einem der vorangegangenen Ansprüche, worin das feste Substrat durch Mikrofabrikation hergestelltes Silizium umfaßt.

16. Vorrichtung nach einem der vorangegangenen Ansprüche, die weiters ein Gerät zur Verwendung in Kombination mit dem Substrat umfaßt, wobei das Gerät umfaßt:
Mittel zum Halten des Substrats;
Fluidzufuhrmittel, die mit der Einlaßöffnung des Substrats ineinanderpassen; und
Pumpmittel, um Fluid durch das Strömungssystem des Substrats, wenn dieses im Haltemittel gehalten wird, hindurchzuleiten.

17. Vorrichtung nach einem der Ansprüche 1 bis 7 und 9 bis 16, worin das Detektionsmittel ein Gerät zur Verwendung in Kombination mit dem Substrat umfaßt, wobei das Gerät umfaßt:
Mittel zum Halten des Substrats; und
optische Mittel zum Betrachten des Inhalts des Meso-Scale-Strömungssystems im Substrat.

18. Vorrichtung nach Anspruch 17, worin das optische Mittel eine Vergrößerungsoptik und eine Videokamera umfaßt und worin das Gerät weiters umfaßt:
einen Kippmechanismus zum manuellen Einstellen des Winkels und der Position der Vorrichtung; und
einen Bildschirm zum Betrachten des Inhalts des Strömungssystems.

19. Vorrichtung nach einem der vorangegangenen Ansprüche, worin das Strömungssystem einen Vergleichsfraktalbereich umfaßt, der einen Vergleich des Probenflusses im Fraktalbereich und im Vergleichsbereich ermöglicht.

20. Verfahren zum Nachweis der Gegenwart eines Analyten in einer Fluidprobe, wobei das Verfahren folgende Schritte umfaßt:
(i) Bereitstellen einer Vorrichtung, umfassend:
ein festes Substrat, das durch Mikrofabrikation so hergestellt ist, daß es definiert:
eine Probeneinlaßöffnung; und
ein Meso-Scale-Durchflußsystem, umfassend:
einen primären Probenströmungskanal, der sich von der Einlaßöffnung weg erstreckt; und
einen Fraktalbereich in Fluidkommunikation mit dem primären Strömungskanal, der mehrere Gabelungen umfaßt, wobei jede Gabelung zu mehreren unterschiedlichen sekundären Strömungskanälen führt, um die Verschlußwirkung des Strömungssystems zu verstärken oder die Viskosität innerhalb des Substrats zu erhöhen,
(ii) Hindurchleiten einer Fluidprobe, von der vermutet wird, daß sie einen Analyten enthält, durch den Fraktalbereich im Meso-Scale-Strömungssystem;
(iii) Bestimmung der Einschränkung oder Blockierung des Flusses der Fluidprobe durch das System hindurch; und
(iv) Korrelieren der nachgewiesenen Strömungseinschränkung oder -blockierung, oder des Fehlens derselben, mit der Gegenwart oder der Abwesenheit eines Analyten in der Probe.

21. Verfahren nach Anspruch 20, worin das Strömungssystem eine Bindungsgruppe umfaßt, die zur Bindung des Analyten in der Probe fähig ist, um die Einschränkung oder Blockierung des Flusses durch das System hindurch zu fördern.

22. Verfahren nach Anspruch 21, worin die Bindungsgruppe auf Teilchen angeordnet ist, die sich mit dem Analyten in der Probe verbinden, um Teilchenagglomeration herbeizuführen, wodurch die Strömungseinschränkung oder -blockierung gefördert wird.

23. Verfahren nach Anspruch 21 oder 22, worin der Analyt eine Zellpopulation in der Probe ist;
die Bindungsgruppe einen Vernetzer für Zellen in der Population umfaßt; und
die Strömungseinschränkung durch mittels Vernetzer herbeigeführte Zellaggregation verursacht wird.

24. Verfahren nach einem der Ansprüche 21 bis 23, worin die Bindungsgruppe innerhalb des Strömungssystems immobilisiert wird.

25. Verfahren nach einem der Ansprüche 20 bis 23, worin die Strömung durch den Aufbau einer makromolekularen Oberflächenschicht auf einer Oberfläche des Strömungssystems eingeschränkt wird.

26. Verfahren nach einem der Ansprüche 20 bis 25, worin der Fraktalbereich bei der in Schritt (i) vorgesehenen Vorrichtung weiters Verbindungen in Fluidkommunikation mit den sekundären Strömungskanälen umfaßt, die zu einem dritten Kanal im Strömungssystem führen.

27. Verfahren nach einem der Ansprüche 20 bis 26, worin in Schritt (iii) die Einschränkung oder Blockierung elektrisch oder optisch bestimmt wird.

28. Verfahren nach einem der Ansprüche 20 bis 27, worin der Fraktalbereich weiters eine Bindungsgruppe zur Bindung einer Komponente der Probe umfaßt.

29. Verfahren nach einem der Ansprüche 20 bis 28, worin der Fraktalbereich Teilchen enthält, die sich mit einer Komponente der Probe verbinden, um Teilchenagglomeration herbeizuführen.

30. Verfahren nach einem der Ansprüche 20 bis 29, worin das in Schritt (i) bereitgestellte Substrat weiters einen Vergleichsbereich in Fluidkommunikation mit der Probeneinlaßöffnung umfaßt; und
worin in Schritt (iii) das Strömen der Probe im Fraktalbereich und im Vergleichsbereich bestimmt und miteinander verglichen wird.

31. Verfahren nach einem der Ansprüche 20 bis 30, worin der Analyt einen replizierbaren prokaryotischen Organismus umfaßt; und
worin in Schritt (iii) die Einschränkung oder Blockierung des Strömens des Organismus durch das Strömungssystem hindurch als Indikator für die Gegenwart des Organismus dient.

32. Verfahren nach einem der Ansprüche 20 bis 31, worin in Schritt (ii) eine Pumpe eingesetzt wird, um die Probe durch das Strömungssystem hindurch zu befördern.

## Revendications

1. Dispositif pour détecter la présence d'un analyte dans un échantillon de fluide, le dispositif comprenant :
un substrat solide microfabriqué pour définir :
un orifice d'entrée de l'échantillon ; et
un système d'écoulement à l'échelle méso, comprenant :
un canal d'écoulement d'échantillon primaire s'étendant à partir dudit orifice d'entrée ; et
une région fractale en communication de fluide avec ledit canal d'écoulement primaire ; et
un moyen pour détecter une propriété d'écoulement d'un échantillon de fluide dans ledit système d'écoulement en tant qu'une indication de la présence d'un analyte dans l'échantillon de fluide ; caractérisé en ce que ladite région fractale comprend des bifurcations multiples, chaque bifurcation menant à plusieurs canaux secondaires distincts d'écoulement pour amplifier l'effet de l'occlusion dudit système d'écoulement ou augmenter la viscosité dans ledit substrat.

2. Dispositif de la revendication 1 où ladite région fractale comprend de plus des jonctions en communication de fluide avec lesdits canaux d'écoulement secondaire menant à un troisième canal d'écoulement dans ledit système d'écoulement à l'échelle méso.

3. Dispositif de la revendication 1 ou de la revendication 2, comprenant de plus un moyen formant pompe pour induire l'écoulement dudit échantillon à travers ledit système d'écoulement à l'échelle méso.

4. Dispositif de la revendication 2 ou de la revendication 3, où ladite région fractale comprend des nombres égaux de bifurcations et de jonctions disposées en série le long de la direction de l'écoulement d'un échantillon à travers ladite région.

5. Dispositif selon l'une quelconque des revendications 2 à 4, où chaque canal secondaire dans ladite région fractale a une aire en section transversale réduite relativement audit canal d'écoulement primaire et audit troisième canal d'écoulement.

6. Dispositif selon l'une quelconque des revendications précédente où ledit moyen pour détecter comprend un moyen pour détecter une propriété d'écoulement du fluide dans ledit troisième canal d'écoulement.

7. Dispositif selon l'une quelconque des revendications 1 à 5, où ledit moyen pour détecter comprend un moyen pour détecter et comparer les propriétés d'écoulement d'un fluide dans ledit canal d'écoulement d'échantillon primaire avec une propriété d'écoulement du fluide dans ledit troisième canal d'écoulement.

8. Dispositif selon l'une quelconque des revendications précédentes où ledit moyen pour détecter comprend :
(a) un moyen de détection électrique ; ou
(b) un moyen de détection magnétique ; ou
(c) un moyen définissant un trajet optique vers ladite région fractale ; ou
(d) un moyen pour détecter la croissance d'un organisme dans ledit système d'écoulement.

9. Dispositif selon l'une quelconque des revendications précédentes, où on détecte la pression d'un fluide ou la conductivité d'un fluide.

10. Dispositif de la revendication 1 comprenant de plus une entité de liaison disposée dans ladite région fractale pour lier un composant dudit échantillon.

11. Dispositif de la revendication 10, où ladite entité de liaison comprend des particules qui se lient à un composant dudit échantillon pour induire une agglomération des particules.

12. Dispositif selon l'une quelconque des revendications précédentes, où ledit substrat définit un certain nombre desdits systèmes d'écoulement.

13. Dispositif selon l'une quelconque des revendications précédentes, où l'échantillon est un échantillon de sperme et où l'écoulement du sperme à travers la région fractale donne une indication de la motilité du sperme.

14. Dispositif de la revendication 13, où ledit substrat est disposé à un angle par rapport à un plan horizontal.

15. Dispositif selon l'une quelconque des revendications précédentes, où ledit substrat solide comprend du silicium microfabriqué.

16. Dispositif selon l'une quelconque des revendications précédentes, comprenant de plus un appareil à utiliser en combinaison avec ledit substrat, ledit appareil comprenant :
un moyen pour maintenir ledit substrat ;
un moyen d'entrée de fluide en relation avec l'orifice d'entrée sur ledit substrat ; et
un moyen formant pompe pour faire passer le fluide à travers le système d'écoulement du substrat quand il est maintenu dans ledit moyen de maintient.

17. Dispositif selon l'une quelconque des revendications 1 à 7 et 9 à 16, où ledit moyen pour détecter comprend un appareil à utiliser en combinaison avec ledit substrat, ledit appareil comprenant :
un moyen pour maintenir ledit substrat ; et
un moyen optique pour voir les contenus dudit système d'écoulement à l'échelle méso dans ledit substrat ;

18. Dispositif de la revendication 17, où ledit moyen optique comprend une optique de grossissement et une caméra vidéo et où ledit appareil comprend de plus :
un mécanisme de basculement pour ajuster manuellement l'angle et l'emplacement du dispositif ; et
un écran vidéo pour voir les contenus dudit système d'écoulement.

19. Dispositif selon l'une quelconque des revendications précédentes où ledit système d'écoulement comprend une région fractale de contrôle permettant une comparaison de l'écoulement dudit échantillon dans ladite région fractale et ladite région de contrôle.

20. Méthode pour détecter la présence d'un analyte dans un échantillon de fluide, la méthode comprenant les étapes de :
(i) prévoir un dispositif comprenant :
un substrat solide microfabriqué pour définir :
un orifice d'entrée de l'échantillon ; et
un système d'écoulement à l'échelle méso comprenant :
un canal d'écoulement d'échantillon primaire s'étendant à partir dudit orifice d'entrée ; et
une région fractale en communication de fluide avec ledit canal d'écoulement primaire, comprenant des bifurcations multiples, chaque bifurcation menant à plusieurs canaux distincts secondaires d'écoulement pour amplifier l'effet de l'occlusion dudit système d'écoulement ou augmenter la viscosité dans ledit substrat ;
(i) Faire passer un échantillon de fluide suspecté de contenir un analyte à travers ladite région fractale dans ledit système d'écoulement à l'échelle méso.
(iii) Détecter le resserrement ou le blocage de l'écoulement de l'échantillon de fluide à travers ledit système ; et
(iv) Mettre le resserrement ou blocage d'écoulement détecté, ou son manque, en corrélation avec la présence ou l'absence d'un analyte dans ledit échantillon.

21. Méthode de la revendication 20, où ledit système d'écoulement comprend une entité de liaison, capable de lier ledit analyte dans ledit échantillon, pour favoriser ledit resserrement ou blocage de l'écoulement à travers ledit système.

22. Méthode de la revendication 21, où ladite entité de liaison est disposée sur des particules qui se lient audit analyte dans ledit échantillon pour induire une agglomération des particules afin de favoriser ainsi ledit resserrement ou blocage de l'écoulement.

23. Méthode de la revendication 1 ou de la revendication 22, où ledit analyte est une population de cellules dans ledit échantillon ;
ladite entité de liaison comprend un agent réticulant des cellules dans ladite population ; et
ledit resserrement de l'écoulement est provoqué par l'agrégation des cellules induite par l'agent réticulant.

24. Méthode selon l'une quelconque des revendications 21 à 23, où ladite entité de liaison est immobilisée dans ledit système d'écoulement.

25. Méthode selon l'une quelconque des revendications 20 à 23, où l'écoulement est resserré par l'accumulation d'une couche de surface macromoléculaire sur une surface dudit système d'écoulement.

26. Méthode selon l'une quelconque des revendications 20 à 25 où ladite région fractale, dans le dispositif prévu à l'étape (i), comprend de plus des jonctions en communication de fluide avec lesdits canaux d'écoulement secondaire menant à un troisième canal dans ledit système d'écoulement.

27. Méthode selon l'une quelconque des revendications 20 à 26 où, à l'étape (iii), le resserrement ou blocage est détecté électriquement ou optiquement.

28. Méthode selon l'une quelconque des revendications 20 à 27, où ladite région fractale comprend de plus une entité de liaison pour la liaison d'un composant dudit échantillon.

29. Méthode selon l'une quelconque des revendications 20 à 28, où ladite région fractale contient des particules qui se lient à un composant dudit échantillon pour induire une agglomération des particules.

30. Méthode selon l'une quelconque des revendications 20 à 29, où ledit substrat prévu à l'étape (i) comprend de plus une région de contrôle en communication de fluide avec ledit orifice d'entrée de l'échantillon ; et
où, à l'étape (iii), l'écoulement dudit échantillon dans ladite région fractale et ladite région de contrôle est détecté et comparé.

31. Méthode selon l'une quelconque des revendications 20 à 30 où l'analyte comprend un organisme procaryote réplicable ; et
où, à l'étape (iii), le resserrement ou blocage de l'écoulement dudit organisme à travers ledit système d'écoulement sert d'indication de la présence dudit organisme.

32. Méthode selon l'une quelconque des revendications 20 à 31 où, à l'étape (ii), une pompe est utilisée pour délivrer ledit échantillon à travers ledit système d'écoulement.
